(19) Europäisches Patentamt
European Patent Office
Office européen des brevets

(11) **EP 1 519 765 B1**

(12) **EUROPÄISCHE PATENTSCHRIFT**

(45) Veröffentlichungstag und Bekanntmachung des Hinweises auf die Patenterteilung:
**09.01.2013 Patentblatt 2013/02**

(51) Int Cl.:
***A61M 1/36*** *(2006.01)*          ***A61M 1/16*** *(2006.01)*

(21) Anmeldenummer: 03732492.8

(22) Anmeldetag: **30.05.2003**

(86) Internationale Anmeldenummer:
**PCT/EP2003/005657**

(87) Internationale Veröffentlichungsnummer:
**WO 2004/004804 (15.01.2004 Gazette 2004/03)**

(54) **VERFAHREN UND VORRICHTUNG ZUR BESTIMMUNG DES RELATIVEN BLUTVOLUMENS WÄHREND EINER EXTRAKORPORALEN BLUTBEHANDLUNG**

METHOD AND DEVICE FOR DETERMINING RELATIVE BLOOD VOLUME DURING AN EXTRACORPOREAL BLOOD TREATMENT

PROCEDE ET DISPOSITIF POUR DETERMINER LE VOLUME SANGUIN RELATIF PENDANT UN TRAITEMENT SANGUIN EXTRACORPOREL

(84) Benannte Vertragsstaaten:
**AT BE BG CH CY CZ DE DK EE ES FI FR GB GR HU IE IT LI LU MC NL PT RO SE SI SK TR**

(30) Priorität: **06.07.2002 DE 10230413**

(43) Veröffentlichungstag der Anmeldung:
**06.04.2005 Patentblatt 2005/14**

(73) Patentinhaber: **Fresenius Medical Care Deutschland GmbH**
**61352 Bad Homburg (DE)**

(72) Erfinder: **ZHANG, Wei**
**97464 Niederwerrn (DE)**

(74) Vertreter: **Oppermann, Frank et al**
**OANDO Oppermann & Oppermann LLP**
**John-F.-Kennedy-Straße 4**
**65189 Wiesbaden (DE)**

(56) Entgegenhaltungen:
EP-A- 0 358 873     DE-A- 10 051 943
DE-C- 19 746 377

EP 1 519 765 B1

**Beschreibung**

Die Erfindung betrifft ein Verfahren zur Bestimmung des relativen Blutvolumens während einer extrakorporalen Blutbehandlung mit einer Blutbehandlungseinrichtung in einem extrakorporalen Blutkreislauf sowie eine Vorrichtung zur Bestimmung des relativen Blutvolumens während einer extrakorporalen Blutbehandlung.

Zur Entfernung von harnpflichtigen Substanzen und zum Flüssigkeitsentzug werden beim akuten und chronischen Nierenversagen verschiedene Verfahren zur apparativen Blutreinigung bzw. Blutbehandlung eingesetzt. Bei der Hämodialyse (HD) überwiegt der diffusive Stofftransport, während bei der Hämofiltration (HF) ein konvektiver Stofftransport über die Membran stattfindet. Eine Kombination aus beiden Verfahren ist die Hämodiafiltration (HDF).

Während der extrakorporalen Blutbehandlung strömt das Blut des Patienten über einen arteriellen Zweig eines Schlauchleitungssystems in eine Blutbehandlungseinrichtung, beispielsweise einen Hämodialysator oder Hämofilter, und strömt von der Blutbehandlungseinrichtung über einen venösen Zweig des Leitungssystems zurück zum Patienten. Das Blut wird mittels einer Blutpumpe, insbesondere Rollenpumpe gefördert, die im arteriellen Zweig des Leitungssystems angeordnet ist. Dem Patienten kann während der extrakorporalen Behandlung Flüssigkeit entzogen werden (Ultrafiltration).

Eine der Hauptkomplikationen bei der extrakorporalen Blutbehandlung stellt ein akuter Blutdruckabfall (Hypotonie) dar, den ein zu hoher bzw. schneller Flüssigkeitsentzug hervorrufen kann.

Es gibt verschiedene Lösungen für dieses Problem. Zum einen sind Blutdruckmonitore bekannt, die eine Änderung des Blutdrucks kontinuierlich überwachen und die Ultrafiltration in Abhängigkeit von der Blutdruckänderung regeln. Zum anderen sind Blutvolumenmonitore bekannt, die das relative Blutvolumen während der Dialysebehandlung messen und eine Regelung der Ultrafiltration in Abhängigkeit vom relativen Blutvolumen vornehmen.

Die DE-C-197 46 377 beschreibt eine Vorrichtung zur Messung des Blutdrucks, die auf der Erfassung der Ausbreitungsgeschwindigkeit oder Laufzeit der sich über das arterielle Gefäßsystem des Patienten fortpflanzenden Pulswellen beruht, die durch dessen Herzkontraktionen erzeugt werden.

Aus der DE-A-40 24 434 ist eine Vorrichtung zur Ultrafiltrationsregelung bekannt, bei der zum Bestimmen des relativen Blutvolumens der Druck im extrakorporalen Kreislauf überwacht wird. Aus der Veränderung des Drucks im Laufe der Blutbehandlung gegenüber dem Druck zu Behandlungsbeginn wird auf die Veränderung des Blutvolumens geschlossen.

Aus der DE 100 51 943 A1 ist ein Verfahren zur nicht-invasiven Blutdruckmessung von Patienten auf der Grundlage von Pulswellenlaufzeiten während einer extrakorporalen Blutbehandlung bekannt.

Der Erfindung liegt die Aufgabe zugrunde, ein Verfahren anzugeben, das die Bestimmung des relativen Brutvolumens während einer extrakorporalen Blutbehandlung ohne größeren apparativen Aufwand erlaubt. Eine weitere Aufgabe der Erfindung ist, eine Vorrichtung zu schaffen, die ohne größeren apparativen Aufwand die Bestimmung des relativen Blutvolumens während einer extrakorporalen Blutbehandlung ermöglicht.

Die Lösung dieser Aufgaben erfolgt erfindungsgemäß mit den Merkmalen der Patentansprüche 1 bzw. 7. Vorteilhafte Ausführungsformen sind Gegenstand der Unteransprüche.

Das erfindungsgemäße Verfahren und die erfindungsgemäße Vorrichtung beruhen auf dem Generieren von Pulswellen in dem extrakorporalen Blutkreislauf, wobei die Ausbreitungsgeschwindigkeit oder Laufzeit der sich im extrakorporalen Kreislauf ausbreitenden Pulswellen gemessen wird. Das relative Blutvolumen wird aus der gemessenen Ausbreitungsgeschwindigkeit bzw. Laufzeit der Pulswellen bestimmt.

Die Erfindung sieht die Bestimmung des relativen Blutvolumens aus dem Verhältnis der Ausbreitungsgeschwindigkeiten oder Laufzeiten der Pulswellen zu zwei verschiedenen Zeitpunkten der Blutbehandlung, insbesondere zu Beginn und während des Verlaufs der Behandlung vor.

Das Blutvolumen kann aus der Ausbreitungsgeschwindigkeit bzw. Laufzeit der Pulswellen bestimmt werden, weil bestimmte Blutbestandteile, z.B. Hämoglobin, Proteine etc. im extrakorporalen Blutkreislauf verbleiben, das Plasmawasser aber entfernt wird. Daher können Änderungen der Konzentration der Blutbestandteile zur Messung der Blutvolumenänderung herangezogen werden.

Das relative Blutvolumen zum Zeitpunkt t ist definiert durch:

$$RBV(t) = \frac{V(t)}{V(0)} \qquad\qquad (1)$$

wobei

  V(0) das Blutvolumen zum Zeitpunkt t = 0, d.h. zu Beginn der Dialysebehandlung, und

V(t) das Blutvolumen zum Zeitpunkt t, d.h. im Verlauf der Behandlung ist.

Da das erfindungsgemäße Verfahren und die erfindungsgemäße Vorrichtung von der ohnehin in den bekannten Dialysemaschinen vorhandenen Druckmessung Gebrauch macht, ist der apparative Aufwand verhältnismäßig gering. Für die Bestimmung des relativen Blutvolumens ist lediglich eine entsprechende Ergänzung der Software für die Mikroprozessorsteuerung der Maschine erforderlich.

In einer bevorzugten Ausführungsform wird die Ausbreitungsgeschwindigkeit oder Laufzeit der Druckpulswellen im extrakorporalen Kreislauf gemessen, die von der Blutpumpe hervorgerufen werden, die im extrakorporalen Kreislauf der bekannten Hämodialysemaschinen angeordnet ist. Bei der Blutpumpe der bekannten Dialysemaschinen handelt es sich im allgemeinen um eine Rollenpumpe, die bei jeder Umdrehung des Pumpenrotors Druckpulse erzeugt.

Die von der Blutpumpe generierten Pulswellen werden vorzugsweise mit einem Drucksensor detektiert, der bei den bekannten Dialysemaschinen im extrakorporalen Kreislauf angeordnet ist.

Zur Steigerung der Genauigkeit kann die Druckmessung vorteilhaft mit einem Drucksensor vorgenommen werden, der ohne Luftsäule in direktem Kontakt am Blutschlauch oder an einer davor vorgesehenen Druckmeßkammer angeordnet ist, durch die die Blutleitung verläuft.

In einer besonders bevorzugten Ausführungsform ist die Blutpumpe im arteriellen Zweig der Blutleitung stromauf der Blutbehandlungseinrichtung und der Drucksensor zum Detektieren der Pulswellen stromab der Blutbehandlungseinrichtung im venösen Zweig der Blutleitung angeordnet. Damit ist die Strecke, über die die Laufzeit zu messen ist, der zwischen Blutpumpe und Drucksensor liegende Teil der Blutleitung.

Wenn der Zeitpunkt, zu dem die Pulswellen von der Blutpumpe generiert werden, nicht bekannt ist, können die von der Blutpumpe generierten Pulswellen mit einem zweiten Drucksensor detektiert werden, der stromauf der Blutbehandlungseinrichtung im arteriellen Zweig der Blutleitung angeordnet ist. Der Zeitpunkt kann aber auch von der Stellung des Pumpenrotors abgeleitet werden, die beispielsweise mit einem Hallsensor erkannt wird. Der Hallsensor kann dabei einen mit dem Rotor sich drehenden Magneten aufweisen, dessen Magnetfeld periodisch eine an dem Stator befindliche Hall-Sonde durchdringt, an der ein entsprechendes elektrisches Spannungssignal abgegriffen werden kann. Im Folgenden wird ein Ausführungsbeispiel einer Dialysemaschine mit einer Vorrichtung zur Bestimmung des relativen Blutvolumens anhand der Zeichnungen näher erläutert.

Es zeigen:

Fig. 1    die wesentlichen Komponenten einer Dialysemaschine mit einer Vorrichtung zur Bestimmung des relativen Blutvolumens in stark vereinfachter schematischer Darstellung und

Fig. 2    den zeitlichen Verlauf der Signale eines arteriellen und venösen Drucksensors zur Bestimmung des Drucks im arteriellen bzw. venösen Zweig der Blutleitung bzw. des Signals eines Hallsensors zur Bestimmung der Stellung des Pumpenrotors.

Die Hämodialysevorrichtung weist einen Dialysator 1 auf, der durch eine semipermeable Membran 2 in eine Blutkammer 3 und eine Dialysierflüssigkeitskammer 4 getrennt ist. Der Einlass der Blutkammer ist mit einem Ende einer arteriellen Blutzuführleitung 5 verbunden, in die eine arterielle Blutpumpe 6 geschaltet ist, während der Auslass der Blutkammer 3 mit einem Ende einer venösen Blutabführleitung 7 verbunden ist, in die eine Tropfkammer 8 geschaltet ist. Blutzutühr- und -abführleitung 5, 7 sind konventionelle Schlauchleitungen, die den arteriellen bzw. venösen Zweig des extrakorporalen Kreislaufs I bilden.

Bei der Blutpumpe 6 handelt es sich um eine konventionelle Rollenpumpe, die mit jeder Umdrehung zwei Druckpulse generiert, die sich über die Blutzufiihrleitung 5, die Blutkammer 3 und die Blutabführleitung 7 im extrakorporalen Blutkreislauf I fortpflanzen. Die Druckpulswellen werden immer dann erzeugt, wenn der Rotor der Rollenpumpe 6 eine bestimmte Stellung einnimmt. Zur Überwachung der Stellung des Pumpenrotors weist die Rollenpumpe 6 einen Hallsensor 33 auf. Das Dialysierflüssigkeitssystem II der Hämodialysemaschine umfasst eine Einrichtung 12 zur Bereitstellung der Dialysierflüssigkeit, die über den ersten Abschnitt 13 einer Dialysierflüssigkeitszuführleitung mit dem Einlass der ersten Kammerhälfte 14a einer Bilanziereinrichtung 15 verbunden ist. Der zweite Abschnitt 16 der Dialysierflüssigkeitszuführleitung verbindet den Auslass der ersten Bilanzierkammerhälfte 14a mit dem Einlass der Dialysierflüssigkeitskammer 4. Der Auslass der Dialysierflüssigkeitskammer 4 ist über den ersten Abschnitt 17a einer Dialysierflüssigkeitsabführleitung mit dem Einlass der zweiten Bilanzierkammerhälfte 14b verbunden. In den ersten Abschnitt 17a der Dialysierflüssigkeitsabführleitung ist eine Dialysierflüssigkeitspumpe 18 geschaltet. Der Auslass der zweiten Bilanzierkammerhälfte 14b ist über den zweiten Abschnitt 17b der Dialysierflüssigkeitsabführleitung mit einem Auslauf 19 verbunden. Stromauf der Dialysierflüssigkeitspumpe 18 zweigt von der Dialysierflüssigkeitsabführleitung 17a eine Ultrafiltratleitung 20 ab, die ebenfalls zu dem Auslauf 19 führt. In die Ultrafiltratleitung 20 ist eine Ultrafiltrationspumpe 21 geschaltet.

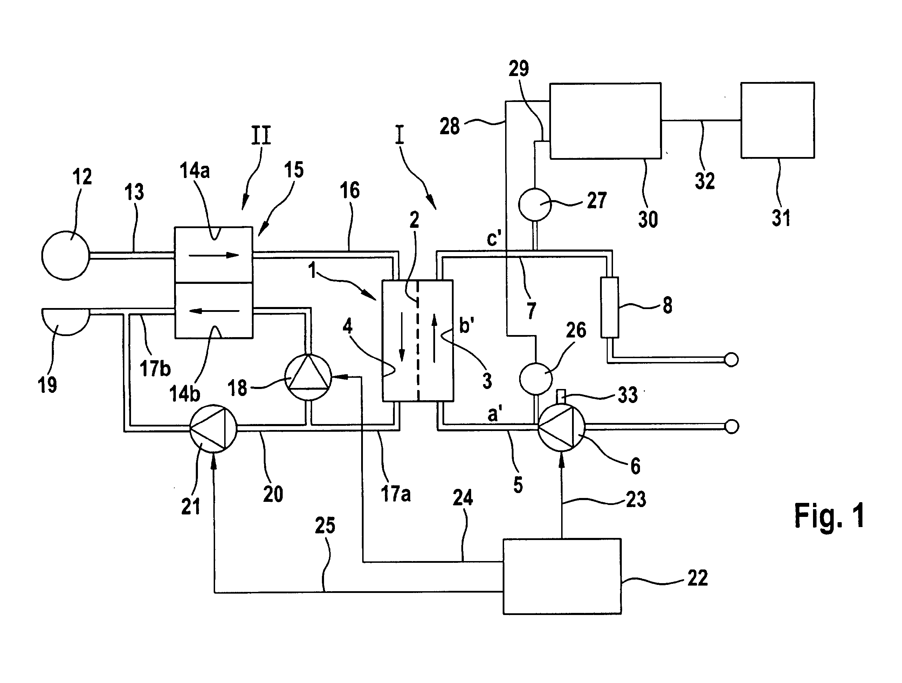

Eine üblicherweise vorhandene zweite Bilanzkammer, die parallel und phasenverschoben zur ersten Bilanzkammer betrieben wird, um einen nahezu konstanten Fluß zu gewährleisten, ist der Einfachheit halber in Fig. 1 nicht gezeigt.

Die Hämodialysemaschine umfasst ferner eine zentrale Steuereinheit 22, die über Steuerleitungen 23 bis 25 mit der Blutpumpe 6, der Dialysierflüssigkeitspumpe 18 und der Ultrafiltrationspumpe 21 verbunden ist.

Während der Hämodialysebehandlung wird die Blutkammer von dem Blut des Patienten und die Dialysierflüssigkeitskammer des Dialysators von der Dialysierflüssigkeit durchströmt. Da die Bilanziereinrichtung 15 in den Dialysierflüssigkeitsweg geschaltet ist, kann nur so viel Dialysierflüssigkeit über die Dialysierflüssigkeitszuführleitung zufliessen, wie Dialysierflüssigkeit über die Dialysierflüssigkeitsabführleitung abfliessen kann. Mit der Ultrafiltrationspumpe 21 kann dem Patienten Flüssigkeit entzogen werden.

Die Hämodialysemaschine weist darüber hinaus eine Vorrichtung zur nicht-invasiven Bestimmung des relativen Blutvolumens während der Dialysebehandlung auf. Diese Vorrichtung macht von verschiedenen Komponenten der Hämodialysemaschine Gebrauch. Daher ist sie Teil der Dialysemaschine. Nachfolgend wird die Vorrichtung zur Bestimmung des relativen Blutvolumens im Einzelnen beschrieben.

Die Vorrichtung zur Bestimmung des relativen Blutvolumens weist einen Drucksensor 26 zum Messen des Drucks in der Blutzuführleitung 5 stromab der Blutpumpe 6 und stromauf der Blutkammer 3 des Dialysators 1 und einen Drucksensor 27 zum Messen des Drucks in der Blutabführleitung 7 stromab der Blutkammer 3 des Dialysators auf. Beide Drucksensoren 26, 27 sind über Signalleitungen 28, 29 mit einer Auswerteinheit 30 verbunden, in der die Signale der Sensoren verarbeitet werden. Diese Auswerteinheit ist Bestandteil der Mikroprozessorsteuerung der Hämodialysemaschine. Aus den gemessenen Druckwerten bestimmt die Auswerteinheit das relative Blutvolumen, das auf einer Anzeigeeinheit 31 angezeigt wird, die über eine Datenleitung 32 mit der Auswerteinheit verbunden ist.

Nachfolgend wird die Funktionsweise der Vorrichtung zur Bestimmung des relativen Blutvolumens RBV beschrieben. Die Bestimmung des relativen Blutvolumens beruht auf der Messung der Laufzeit der von der Blutpumpe 6 generierten Pulswellen, die sich im extrakorporalen Blutkreislauf I ausbreiten. Die Messstrecke L setzt sich aus den Teilen der Blutleitung und der Blutkammer zwischen arteriellem und venösem Drucksensor 26, 27 zusammen. Diese Strecke L ist in Fig. 1 mit a', b' und c' bezeichnet.

Der theoretische Zusammenhang zwischen der Pulswellenlaufzeit und dem RBV wird wie folgt hergeleitet. In einer inkompressiblen Flüssigkeit, die sich in einem elastischen zylindrischen Rohr mit der Querschnittfläche A befindet, wird die Ausbreitungsgeschwindigkeit c einer longitudinalen Druckwelle gegeben durch:

$$c = \sqrt{\frac{Adp}{\rho dA}} \qquad (2)$$

wobei

c     Pulswellengeschwindigkeit
$\rho$     Dichte der Flüssigkeit
dp     Druckänderung
dA     Flächenänderung

Bei der Dialysebehandlung beträgt die Laufzeit PTT ("Pulse Transit Time") über den Teil des Blutschlauchsystems (Messstrecke) mit der Gesamtlänge L zwischen dem vorzugsweise unmittelbar stromab der Blutpumpe angeordneten arteriellen Drucksensor bzw. der Blutpumpe und dem venösen Drucksensor:

$$PTT = \frac{L}{c} = L\sqrt{\frac{\rho}{A} \cdot \frac{dA}{dp}} \qquad (3)$$

Aus Gleichung (3) ergibt sich:

$$PTT(t_0) = L\sqrt{\rho(t_0)\left(\frac{dA\,/\,A(t_0)}{dp}\right)_{t_0}} \qquad (4)$$

$$PTT(t) = L\sqrt{\rho(t)\left(\frac{dA\,/\,A(t)}{dp}\right)_{t}} \qquad (5)$$

wobei

PTT($t_0$)    Laufzeit zum Zeitpunkt $t_0$
PTT(t)    Laufzeit zum Zeitpunkt t

Mit Gleichung (4) und (5) erhält man:

$$\frac{PTT(t)}{PTT(t_0)} = \sqrt{\frac{\rho(t)}{\rho(t_0)}\frac{\left(\frac{dA\,/\,A(t)}{dp}\right)_{t}}{\left(\frac{dA\,/\,A(t_0)}{dp}\right)_{t_0}}} \qquad (6)$$

$$\left(\frac{PTT(t)}{PTT(t_0)}\right)^2 = \frac{\rho(t)}{\rho(t_0)}K(P) \qquad (7)$$

$$K(P) = \left(\frac{dA\,/\,A(t)}{dp}\right)_{t} \Big/ \left(\frac{dA\,/\,A(t_0)}{dp}\right)_{t_0} \qquad (8)$$

Hierbei bezeichnet K(P) das Verhältnis der Dehnungsgrösse des Schlauchs zum Zeitpunkt t und $t_0$.
Die Massendichte des Blutes ist mit dem Verhältnis des Massenanteils des Proteins und Wassers im Blut zum gesamten Blutvolumen definiert durch:

$$\rho(t_0) = \frac{m_{protein}(t_0) + m_{water}(t_0)}{V(t_0)} \qquad (9)$$

$$\rho(t) = \frac{m_{protein}(t) + m_{water}(t)}{V(t)} \qquad (10)$$

wobei

$\rho(t_0)$          Massendichte des Blutes zum Zeitpunkt $t_0$
$p(t)$          Massendichte des Blutes zum Zeitpunkt $t$
$V(t_0)$          Blutvolumen zum Zeitpunkt $t_0$
$V(t)$          Blutvolumen zum Zeitpunkt $t$
$m_{protein}(t_0)$          Masse der Proteine in $V(t_0)$ zum Zeitpunkt $t_0$
$m_{protein}(t)$          Masse der Proteine in $V(t_0)$ zum Zeitpunkt $t$
$m_{water}(t_0)$          Masse des Wassers in $V(t_0)$ zum Zeitpunkt $t_0$
$m_{water}(t)$          Masse des Wassers in $V(t_0)$ zum Zeitpunkt $t$

Da die Membran eines Dialysators für den überwiegenden Teil der Blutproteine nicht durchlässig ist, bleibt der Blutproteingehalt während der Hämodialyse annähernd konstant, d.h. $m_{protein}(t) = m_{protein}(t_0)$. Aus Gleichung (9), (10) und (1) ergibt sich:

$$\frac{\rho(t)}{\rho(t_0)} = \frac{1}{RBV(t)}\left(1 - \frac{m_{water}(t_0) - m_{water}(t)}{m_{protein}(t_0) + m_{water}(t_0)}\right) \qquad (11)$$

Mit $m_{water}(t_0) - m_{water}(t) = V(t_0) \cdot [1 - RBV(t)]$. $\rho_w$ lässt sich Gleichung (11) in der Form

$$\frac{\rho(t)}{\rho(t_0)} = \frac{1}{RBV(t)}\left(1 - \frac{\rho_w}{\rho(t_0)} + RBV(t)\frac{\rho_w}{\rho(t_0)}\right) \qquad (12)$$

schreiben, wobei $\rho_w$ die Massendichte von Wasser bezeichnet.
Mit Gleichung (7) und (12) erhält man

$$\left(\frac{PTT(t)}{PTT(t_0)}\right)^2 = \frac{1}{RBV(t)}\left(1 - \frac{\rho_w}{\rho(t_0)} + RBV(t)\frac{\rho_w}{\rho(t_0)}\right)K(P) \qquad (13)$$

Die Lösung dieser Gleichung lautet

$$RBV(t) = \frac{\left(1 - \dfrac{\rho_w}{\rho(t_0)}\right)K(P)}{\left(\dfrac{PTT(t)}{PTT(t_0)}\right)^2 - \dfrac{\rho_w}{\rho(t_0)}K(P)} \qquad (14)$$

Wenn das Schlauchsystem elastisch ist und es innerhalb des Proportionalitätsbereichs (Elastizitätsbereich) bei der Behandlung bleibt, ist nach dem Hookeschen Gesetz $K(P)=1$. Daraus ergibt sich:

$$RBV(t) = \frac{1 - \dfrac{\rho_w}{\rho(t_0)}}{\left(\dfrac{PTT(t)}{PTT(t_0)}\right)^2 - \dfrac{\rho_w}{\rho(t_0)}} \qquad (15)$$

Gleichung (15) zeigt, dass das relative Blutvolumen RBV(t) eine Funktion des Verhältnisses der Laufzeiten und der Blutdichte zum Zeitpunkt $t_0$ ist. Unter der Annahme, dass die Blutdichte vor der Dialysebehandlung für alle Patienten annähernd gleich ist, hängt RBV(t) nur vom Verhältnis der Laufzeiten ab.

Wenn die Elastizität des Schlauchs allerdings vom Druck im Schlauch abhängt, insbesondere wenn ein nicht linearer Zusammenhang zwischen der Elastizität und dem Druck besteht, kann eine Kennkurve für K(P) verwendet werden.

Zu Beginn der Dialysebehandlung bestimmt die Auswerteinheit 30 die Laufzeit $PTT(t_0)$ zum Zeitpunkt $t_0$. Dieser Wert wird in einem Speicher gespeichert. In diesen Speicher werden auch die Werte für die Massendichte $\rho_w$ von Wasser und die Massendichte $\rho(t_0)$ des Blutes zu Beginn der Dialysebehandlung eingelesen. Diese Werte werden als Konstanten angenommen. Sie können extern eingegeben oder fest vorgegeben sein.

Zur Bestimmung der Laufzeit $PTT(t_0)$ wird die Zeit gemessen, die eine Pulswelle benötigt, um vom arteriellen Drucksensor 26 zum venösen Drucksensor 27 zu gelangen.

Auch wenn die Meßstrecke a'+b'+c' in Fig. 1 eine lange Meßzeit erlaubt, ist zu berücksichtigen, dass sich längs dieser Strecke Elemente mit verschiedener Elastizität befinden. So haben beispielsweise Dialysator und Blutschlauch bezüglich der Elastizität unterschiedliche Eigenschaften. Zur Vermeidung von Störeinflüssen kann daher auch nur über eine Meßstrecke längs des Blutschlauches stromauf oder stromab des Dialysators gemessen werden. Dann sind entweder stromab der Blutpumpe ein arterieller Drucksensor zur Messung der Laufzeit zwischen Pumpe und arteriellem Drucksensor oder zwei venöse Drucksensoren zur Messung der Laufzeit zwischen den beiden venösen Sensoren vorzusehen.

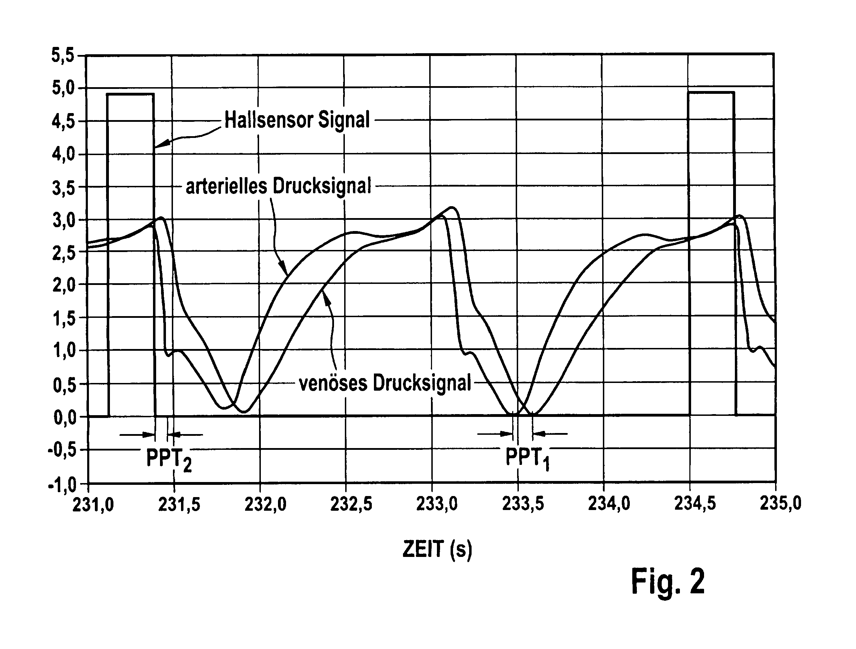

Fig. 2 zeigt den zeitlichen Verlauf der Drucksignale der Drucksensoren 26, 27. Deutlich ist zu erkennen, dass die Pulswelle erst an dem arteriellen und dann an dem venösen Drucksensor eintrifft. Die Laufzeit über die Messstrecke L zwischen arteriellem und venösem Drucksensor ist in Fig. 2 mit $PTT_1$ bezeichnet. Um eine besonders lange Messstrecke zu haben, sollte der arterielle Drucksensor 26 unmittelbar stromab der Blutpumpe 6 und der venöse Drucksensor 27 möglichst weit stromab der Blutkammer 3 in der Blutleitung angeordnet sein.

Während der Dialysebehandlung bestimmt die Auswerteinheit 30 fortlaufend die Laufzeit PTT (t) der Pulswellen und berechnet nach Gleichung (15) fortlaufend das relative Blutvolumen RBV(t).

Unter der Annahme eines nicht linearer Zusammenhang zwischen der Elastizität und dem Druck, wird eine Kennkurve für K(p) im Speicher abgelegt. Dann erfolgt die Berechnung des relativen Blutvolumens nach Gleichung (14).

Eine alternative Ausführungsform sieht nur einen venösen Drucksensor 27 in der Blutabführleitung 7 vor. Der arterielle Drucksensor 26 in der Blutzuführleitung 5 ist grundsätzlich nicht erforderlich. Anstelle des arteriellen Drucksensors kann das Auftreten der Pulswellen mit dem Hallsensor 33 der Blutpumpe detektiert werden.

Fig. 2 zeigt das Hallsignal des Sensors 33. Deutlich ist zu erkennen, dass die negativen Flanken des Hall- und Drucksignals übereinstimmen. Die Laufzeit über die Strecke zwischen Blutpumpe und venösem Drucksensor ist in Fig. 2 mit $PTT_2$ bezeichnet. Da der Magnet auf dem Rotor der Blutpumpe nur zu einem Signal pro Umdrehung führt und der Rotor zwei okkludierende Rollen aufweist, tritt das Hallsignal nur mit der halben Frequenz gegenüber dem Drucksignal auf.

**Patentansprüche**

1. Verfahren zur Bestimmung des relativen Blutvolumens während einer extrakorporalen Blutbehandlung mit einer Blutbehandlungseinrichtung in einem extrakorporalen Blutkreislauf, der einen zu der Blutbehandlungseinrichtung führenden arteriellen Zweig einer Blutleitung und einen von der Blutbehandlungseinrichtung abgehenden venösen Zweig der Blutleitung aufweist, **dadurch gekennzeichnet, dass** Pulswellen in dem extrakorporalen Blutkreislauf generiert werden, wobei die Ausbreitungsgeschwindigkeit oder Laufzeit der sich im extrakorporalen Kreislauf ausbreitenden Pulswellen gemessen und das relative Blutvolumen RBV(t) aus dem Verhältnis der Laufzeiten oder Ausbreitungsgeschwindigkeiten der Pulswellen zu zwei verschiedenen Zeitpunkten t, $t_0$ der Blutbehandlung bestimmt wird.

**2.** Verfahren nach Anspruch 1, **dadurch gekennzeichnet, dass** die Ausbreitungsgeschwindigkeit oder Laufzeit der von einer Blutpumpe im extrakorporalen Kreislauf generierten Pulswellen gemessen wird.

**3.** Verfahren nach Anspruch 1 oder 2, **dadurch gekennzeichnet, dass** die von der Blutpumpe generierten Pulswellen mit einem im extrakorporalen Kreislauf angeordneten Drucksensor detektiert werden.

**4.** Verfahren nach Anspruch 3, **dadurch gekennzeichnet, dass** die Blutpumpe im arteriellen Zweig der Blutleitung stromauf der Blutbehandlungseinrichtung und der Drucksensor zum Detektieren der Pulswellen stromab der Blutbehandlungseinrichtung im venösen Zweig der Blutleitung angeordnet ist.

**5.** Verfahren nach Anspruch 4, **dadurch gekennzeichnet, dass** die von der Blutpumpe generierten Pulswellen mit einem zweiten Drucksensor detektiert werden, der stromauf der Blutbehandlungseinrichtung im arteriellen Zweig der Blutleitung angeordnet ist.

**6.** Verfahren nach Anspruch 5, **dadurch gekennzeichnet, dass** das relative Blutvolumen RBV(t) aus der zeitlichen Änderung der Laufzeit der Pulswellen nach der folgenden Gleichung berechnet wird:

$$RBV(t) = \frac{1 - \dfrac{\rho_w}{\rho(t_0)}}{\left(\dfrac{PTT(t)}{PTT(t_0)}\right)^2 - \dfrac{\rho_w}{\rho(t_0)}}$$

wobei

PTT(t) und PTT($t_0$) die Laufzeit der Pulswellen über einen Streckenabschnitt des extrakorporalen Blutkreislaufs mit einer vorgegebenen Länge L zum Zeitpunkt t und $t_0$ und $\rho_w$ die Massendichte von Wasser und $\rho(t_0)$ die Massendichte des Blutes zu Beginn der Blutbehandlung ist.

**7.** Vorrichtung zur Bestimmung des relativen Blutvolumens während einer extrakorporalen Blutbehandlung mit einem extrakorporalen Blutkreislauf (I), der einen zu einer Blutbehandlungseinrichtung (1) führenden arteriellen Zweig (5) einer Blutleitung (5, 7) und einen von der Blutbehandlungseinrichtung abgehenden venösen Zweig (7) der Blutleitung (5, 7) aufweist, mit

Mitteln (26,27, 30) zum Messen der Ausbreitungsgeschwindigkeit oder Laufzeit der sich in dem extrakorporalen Kreislauf ausbreitenden ulswellen, und mit

Mittel (6) zum Generieren von Pulswellen in dem extrakorporalen Kreislauf (I), **gekennzeichnet durch**

Mittel (30) zum Bestimmen des relativen Blutvolumens, die derart ausgebildet sind, dass das relative Blutvolumen RBV(t) aus dem Verhältnis der Laufzeiten oder Ausbreitungsgeschwindigkeiten der Pulswellen zu zwei verschiedenen Zeitpunkten t, $t_0$ der Blutbehandlung bestimmbar ist.

**8.** Vorrichtung nach Anspruch 7, **dadurch gekennzeichnet, dass** im extrakorporalen Kreislauf (I) eine Blutpumpe (6) angeordnet ist, wobei die Mittel (26, 27, 30) zum Messen der Laufzeit oder Ausbreitungsgeschwindigkeit der Pulswellen derart ausgebildet sind, dass die Ausbreitungsgeschwindigkeit oder Laufzeit der von der Blutpumpe (6) im extrakorporalen Kreislauf generierten Pulswellen gemessen wird.

**9.** Vorrichtung nach Anspruch 7 oder 8, **dadurch gekennzeichnet, dass** im extrakorporalen Kreislauf (I) ein Drucksensor (27) zum Detektieren der von der Blutpumpe (6) generierten Pulswellen angeordnet ist.

**10.** Vorrichtung nach Anspruch 9, **dadurch gekennzeichnet, dass** die Blutpumpe (6) im arteriellen Zweig (5) der Blutleitung (5, 7) stromauf der Blutbehandlungseinrichtung (1) und der Drucksensor (27) zum Detektieren der Pulswellen stromab der Blutbehandlungseinrichtung im venösen Zweig (7) der Blutleitung (5, 7) angeordnet ist.

**11.** Vorrichtung nach Anspruch 10, **dadurch gekennzeichnet, dass** ein zweiter Drucksensor (26) zum Detektieren der Pulswellen stromauf der Blutbehandlungseinrichtung (1) im arteriellen Zweig der Blutleitung (5, 7) angeordnet ist.

**12.** Vorrichtung nach Anspruch 11, **dadurch gekennzeichnet, dass** die Mittel (30) zum Bestimmen des relativen Blutvolumens derart ausgebildet sind, dass das relative Blutvolumen RBV(t) aus der zeitlichen Änderung der Laufzeiten der Pukswellen nach der folgenden Gleichung berechnet wird,

$$RBV(t) = \frac{1 - \dfrac{\rho_w}{\rho(t_0)}}{\left(\dfrac{PTT(t)}{PTT(t_0)}\right)^2 - \dfrac{\rho_w}{\rho(t_0)}}$$

wobei PTT(t) und PTT(t$_0$) die Laufzeit der Pulswellen über einen Streckenabschnitt des extrakorporalen Blutkreislaufs mit einer vorgegebenen Länge L zum Zeitpunkt t und t$_0$ und $\rho_w$ die Massendichte von Wasser und $\rho(t_0)$ die Massendichte des Blutes zu Beginn der Blutbehandlung ist.

## Claims

1. Method of determining relative blood volume during extra-corporeal blood treatment, by a blood treatment device, in an extra-corporeal blood circuit which has an arterial section of a blood line, which arterial section runs to the blood treatment device, and a venous section of the blood line, which venous section runs from the blood treatment device, **characterised in that** pulse waves are generated in the extra-corporeal blood circuit, the velocity of propagation or transit time of the pulse waves propagating in the extra-corporeal circuit being measured and the relative blood volume RBV(t) being determined from the ratio of the transit times or velocities of propagation of the pulse waves at two different times t, to in the blood treatment.

2. Method according to claim 1, **characterised in that** the velocity of propagation or transit time of the pulse waves generated by an blood pump in the extra-corporeal circuit is measured.

3. Method according to claim 1 or 2, **characterised in that** the pulse waves generated by the blood pump are detected by a pressure sensor arranged in the extra-corporeal circuit.

4. Method according to claim 3, **characterised in that** the blood pump is arranged in the arterial section of the blood line, upstream of the blood treatment device, and the pressure sensor for detecting the pulse waves is arranged in the venous section of the blood line, downstream of the blood treatment device.

5. Method according to claim 4, **characterised in that** the pulse waves generated by the blood pump are detected by a second pressure sensor which is arranged in the arterial section of the blood line, upstream of the blood treatment device.

6. Method according to claim 5, **characterised in that** the relative blood volume RBV(t) is calculated from the change over time in the transit time of the pulse waves, from the following equation:

$$RBV(t) = \frac{1 - \dfrac{\rho_W}{\rho(t_0)}}{\left(\dfrac{PTT(t)}{PTT(t_0)}\right)^2 - \dfrac{\rho_W}{\rho(t_0)}}$$

where
PTT(t) and PTT(t$_0$) are the transit times at times t and to of the pulse waves over a portion of a preset length L of the distance covered by the extra-corporeal blood circuit, $\rho_w$ is the mass density of water, and $\rho(t_0)$ is the mass density of the blood at the beginning of the blood treatment.

7. Arrangement for determining relative blood volume during extra-corporeal blood treatment, having an extra-corporeal blood circuit (I) which has an arterial section (5) of a blood line (5, 7), which arterial section (5) runs to a blood treatment device (1), and a venous section (7) of the blood line (5, 7), which venous section (7) runs from the blood treatment device, having means (26, 27, 30) for measuring the velocity of propagation or transit time of pulse waves propagating in the extra-corporeal circuit, and having means (6) for generating pulse waves in the extra-corporeal circuit (I), **characterised by**
means (30) for determining the relative blood volume which are so designed that the relative blood volume RBW(t) can be determined from the ratio of the transit times or velocities of propagation of the pulse waves at two different times t, to in the blood treatment.

8. Arrangement according to claim 7, **characterised in that** a blood pump (6) is arranged in the extra-corporeal circuit (I), the means (26, 27, 30) for measuring the transit time or velocity of propagation of the pulse waves being so designed that the velocity of propagation or transit time of the pulse waves generated by the blood pump (6) in the extra-corporeal circuit is measured.

9. Arrangement according to claim 7 or 8, **characterised in that** a pressure sensor (27) to detect the pulse waves generated by the blood pump (6) is arranged in the extra-corporeal circuit (I).

10. Arrangement according to claim 9, **characterised in that** the blood pump (6) is arranged in the arterial section (5) of the blood line (5, 7), upstream of the blood treatment device (1), and the pressure sensor (27) for detecting the pulse waves is arranged in the venous section (7) of the blood line (5, 7), downstream of the blood treatment device.

11. Arrangement according to claim 10, **characterised in that** a second pressure sensor (26) for detecting the pulse waves is arranged in the arterial section of the blood line (5, 7), upstream of the blood treatment device (1).

12. Arrangement according to claim 11, **characterised in that** that the means (30) for determining the relative blood volume are so designed that the relative blood volume RBV(t) is calculated from the change over time in the transit times of the pulse waves, from the following equation:

$$RBV(t) = \frac{1 - \frac{\rho_w}{\rho(t_0)}}{\left(\frac{PTT(t)}{PTT(t_0)}\right)^2 - \frac{\rho_w}{\rho(t_0)}}$$

where
PTT(t) and PTT($t_0$) are the transit times at times t and to of the pulse waves over a portion of a preset length L of the distance covered by the extra-corporeal blood circuit, $\rho_w$ is the mass density of water, and $\rho(t_0)$ is the mass density of the blood at the beginning of the blood treatment.

## Revendications

1. Procédé de détermination du volume sanguin relatif pendant un traitement sanguin extracorporel avec une installation de traitement sanguin dans une circulation sanguine extracorporelle, qui présente une ramification artérielle d'une conduite de sang conduisant à l'installation de traitement sanguin et une ramification veineuse de la conduite de sang partant de l'installation de traitement sanguin, **caractérisé en ce que** des ondes d'impulsion sont générées dans la circulation sanguine extracorporelle, la vitesse de diffusion ou le temps de parcours des ondes d'impulsion se diffusant dans la circulation extracorporelle étant mesuré(e) et le volume sanguin relatif RBV(t) étant déterminé à partir du rapport des temps de parcours ou des vitesses de diffusion des ondes d'impulsion à deux temps différents t, $t_0$ du traitement sanguin.

2. Procédé selon la revendication 1, **caractérisé en ce que** la vitesse de diffusion ou le temps de parcours des ondes d'impulsion générées par une pompe de sang dans la circulation extracorporelle est mesurée.

3. Procédé selon la revendication 1 ou 2, **caractérisé en ce que** les ondes d'impulsion générées par la pompe de sang sont détectées avec un capteur de pression disposé dans la circulation extracorporelle.

4. Procédé selon la revendication 3, **caractérisé en ce que** la pompe de sang dans la ramification artérielle de la conduite de sang est disposée en amont de l'installation de traitement sanguin et le capteur de pression pour détecter les ondes d'impulsion est disposé en aval de l'installation de traitement sanguin dans la ramification veineuse de la conduite de sang.

**5.** Procédé selon la revendication 4, **caractérisé en ce que** les ondes d'impulsion générées par la pompe de sang sont détectées avec un deuxième capteur de pression qui est disposé en amont de l'installation de traitement sanguin dans la ramification artérielle de la conduite de sang.

**6.** Procédé selon la revendication 5, **caractérisé en ce que** le volume sanguin relatif RBV(t) est calculé à partir de la modification temporelle du temps de parcours des ondes d'impulsion selon l'équation suivante :

$$RBV(t) = \frac{1 - \dfrac{\rho_w}{\rho(t_0)}}{\left(\dfrac{PTT(t)}{PTT(t_0)}\right)^2 - \dfrac{\rho_w}{\rho(t_0)}}$$

où
PTT(t) et PTT(t$_0$) sont le temps de parcours des ondes d'impulsion sur un segment de parcours de la circulation sanguine extracorporelle avec une longueur prédéterminée L par rapport aux temps t et t$_0$ et $\rho_w$ est la densité de masse de l'eau et $\rho(t_0)$ est la densité de masse du sang au début du traitement sanguin.

**7.** Dispositif de détermination du volume sanguin relatif pendant un traitement sanguin extracorporel avec une circulation sanguine extracorporelle (I) qui présente une ramification artérielle (5) d'une conduite de sang (5,7) conduisant à l'installation de traitement sanguin et une ramification veineuse (7) de la conduite de sang (5, 7) partant de l'installation de traitement sanguin, avec
des moyens (26, 27, 30) de mesure de la vitesse de diffusion ou du temps de parcours des ondes d'impulsion se diffusant dans la circulation extracorporelle, et avec des moyens (6) de génération d'ondes d'impulsion dans la circulation extracorporelle (I)
**caractérisé par**
des moyens (30) pour déterminer le volume sanguin relatif, qui sont conçus de telle sorte que le volume sanguin relatif RBV(t) peut être déterminé à partir du rapport des temps de parcours ou des vitesses de diffusion des ondes d'impulsion aux deux temps différents t, t$_0$ de traitement sanguin.

**8.** Dispositif selon la revendication 7, **caractérisé en ce que**, dans la circulation extracorporelle (I), une pompe de sang (6) est disposée, les moyens (26, 27, 30) étant conçus pour mesurer le parcours ou la vitesse de diffusion des ondes d'impulsion de telle sorte que la vitesse de diffusion ou le temps de parcours des ondes d'impulsion générées par la pompe de sang (6) dans la circulation extracorporelle soit mesurée.

**9.** Dispositif selon la revendication 7 ou 8, **caractérisé en ce que**, dans la circulation extracorporelle (I) est disposé un capteur de pression (27) pour détecter les ondes d'impulsion générées par la pompe de sang (6).

**10.** Dispositif selon la revendication 9, **caractérisé en ce que** la pompe de sang (6) dans la ramification artérielle (5) de la conduite de sang (5, 7) est disposée en amont du dispositif de traitement du sang (1) et le capteur de pression (27) pour la détection des ondes d'impulsion est disposé en aval de l'installation de traitement sanguin dans la ramification veineuse (7) de la conduite de sang (5, 7).

**11.** Dispositif selon la revendication 10, **caractérisé en ce qu'**un deuxième capteur de pression (26) pour la détection des ondes d'impulsion est disposé en amont de l'installation de traitement sanguin (1) dans la ramification artérielle de la conduite de sang (5, 7).

**12.** Dispositif selon la revendication 11, **caractérisé en ce que** le moyen (30) pour déterminer le volume sanguin relatif est conçu de telle sorte que le volume sanguin relatif RBV(t) est calculé à partir de la modification temporelle du temps de parcours des ondes d'impulsion selon l'équation suivante :

$$RBV(t) = \frac{1 - \dfrac{\rho_w}{\rho(t_0)}}{\left(\dfrac{PTT(t)}{PTT(t_0)}\right)^2 - \dfrac{\rho_w}{\rho(t_0)}}$$

où

PTT(t) et PTT($t_0$) sont le temps de parcours des ondes d'impulsion sur un segment de parcours de la circulation sanguine extracorporelle avec une longueur prédéterminée L par rapport aux temps t et $t_0$ et $\rho_w$ est la densité de masse de l'eau et $\rho(t_0)$ est la densité de masse du sang au début du traitement sanguin.

Fig. 1

Fig. 2

EP 1 519 765 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19746377 C
- DE 4024434 A

- DE 10051943 A1